# EUROPÄISCHE PATENTSCHRIFT

(11) **EP 0 514 508 B1**
(45) Veröffentlichungstag und Bekanntmachung des Hinweises auf die Patenterteilung: **15.03.1995**
(21) Anmeldenummer: 91920013.9
(22) Anmeldetag: 27.11.1991
(51) Int. Cl.: A61K 35/78, C07G 17/00

(54) **Extrakt aus Calotropis procera zur Behandlung von angeborene Syphilis**
Extract of Calotropis procera for the treatment of congenital syphilis
Extrait de Calotropis procera pour le traitement du syphilis congénital

(30) Priorität: 27.11.1990 CH 3744/90
(43) Veröffentlichungstag der Anmeldung: 25.11.1992
(73) Patentinhaber: MRAK, Magdalena, CH-5200 Brugg (CH)
(72) Erfinder: MRAK, Magdalena, CH-5200 Brugg (CH)
(74) Vertreter: Révy von Belvárd, Peter
(86) Internationale Anmeldenummer: CH9100241
(87) Internationale Veröffentlichungsnummer: WO9209295

(56) Entgegenhaltungen:
- Biological Abstracts, Band 84, Nr. 1, 1987 (Philadelphia, PA, US) S.K.Bhatnagar et al.: "Effects of 50 percent ethanol extract of Calotropis proceraAIT.f. on ulcers caused by assorted types of carcinoma", siehe Seite AB-739,Zusammenfassung 6510, & J. Econ. Taxon. Bot. 8(2): 489-490, 1986
- Biological Abstracts, Band 82, Nr. 4, 1986 (Philadelphia, PA, US) R. Ribeiro et al.:"Acute-antihypersensitive effect in conscious rats produced by somemedicinal plants used in the state of Sao Paulo (Brazil)", Page AB-37, Abst ract 37351
- Biological Abstracts, Band 87, Nr. 3, 1989 (Philadelphia, PA, US) R. Ribeiro etal.: "Acute diuretic effects in conscious rats produced by some medicinalplants used in the state of Sao Paulo (Brazil)", siehe Seite AB-1060,Zusammenfassung 32970, & J. Ethnopharmacol., 24(1), 19-30, 1988
- Biological Abstracts, Band 91, Nr. 11, 1. Juni 1991 (Philadelphia, PA, US) I.Basualdo et al.: "Medicinal plants of Paraguay: Underground organs", sieheSeite AB-951, Zusammenfassung 123003, & Econ. Bot., 45(1), 86-96, 1991

## Beschreibung

Die Erfindung betrifft die Verwendung von Calotropis procera für die Herstellung eines Extraktes zur Behandlung angeborener Syphilis.

Verschiedentlich, und besonders in tropischen Ländern, ist die angeborene Syphilis (congenital syphilis) sowie die "bejel" genannte Form dieser Krankheit stark verbreitet. Sie wird insbesondere über die Plazenta von einer infizierten Frau auf das Kind übertragen. Sie führt zu einem harten Gewschwür nächst der infizierten Stelle, zu Schwellungen der Lymphknoten, allenfalls Fieber, Ausschlägen und erst nach geraumer Zeit, manchmal nach Jahren zu tumorartigen Massen, in jedem Falle auch zu Schmerzen der betroffenen Person.

Zur Heilung dieser Krankheit wird im allgemeinen Penicillin eingesetzt, doch ist die Krankheit - trotz der anfänglichen grossen Erfolge - wieder im Zunehmen begriffen, wobei es überdies bei manchen Personen zu allergischen Reaktionen bei Verabreichung von Penicillin kommt. Es besteht somit ein Bedarf an einem Mittel, das allergische Reaktionen und die Bilodung resistenter Stämme vermeidet, jedenfalls aber als alternatives Mittel eingesetzt werden kann, und dies ist die der Erfindung zugrundeliegende Aufgabe.

Diese Aufgabe wird erfindungsgemäss durch die Merkmale des Anspruches 1 gelöst.

### Die Pflanzen

Die wichtigste aller Pflanzen ist der Sodomsapfel oder Oscherstrauch, Calotropis procera. Diese Pflanze gehört zu den Ascepiadaceae, den Seidenpflanzengewächsen, und ist von West-afrika bis Hinterindien verbreitet, wo sie in fünf Arten in trockenen Steppen und an wüstenhaften Standorten oft massenhaft auftritt. Die im Grunde verholzte Pflanze erreicht eine Höhe von mehr als 4 m und kann so bis zu einem kleinen Baum heranwachsen. Die Pflanze Calotropis procera ist an sich bekannt. Ihre Inhaltsstoffe, vor allem das Calotropin, sind den Herzglykosiden verwandt. Biol. Abstracts, Vol. 84 (1987), Ref No. 6510, berichtet allerdings auch von den Wirkungsen eines 50%-igen Extrakts, das 1-9 Tage lang bei krebsartigen Geschwüren verabreicht wurde und in 60% der Fälle zu einer Rückbildung führte.

Insoferne ist die der Erfindung zugrundeliegende Erkenntnis überraschend, dass sich ein solches Extrakt dieser Pflanze auch beim oben angegebenen Krankheitsbild bewährt, wenn auch die Therapie länger dauert. In üblicher Weise werden für den Extrakt die grünen Stämme mit den grünen Äpfeln, aber auch die junge Pflanze mit den Wurzeln benutzt, wobei zweckmässig jeder Teil einzeln präpariert wird.

Wie aber aus der Literatur eben hervorgeht, ergeben sich bei einer reinen Therapie mit Calotropis procera auch Probleme durch seine Herzglykosidwirkung, die hier natürlich eine unerwünschte Nebenwirkung darstellt. Dem kann einerseits durch Verringerung der Dosis begegnet werden, doch wurde gefunden, dass der Extrakt der sog. Schmetterlingsblume oder Hedychie, ein Ingwergewächs, eine wertvolle Ergänzung bietet.

Die Gattung Hedychium umfasst 50 Arten, von denen die meisten in Ostindien und je zwei auf den Philippinen und auf Madagaskar verbreitet sind. Die wichtigste darunter ist Hedychium coronarium, welche als Heilpflanze bekannt ist [vgl. Biol. Abstracts, Vol. 82 (1986), Ref.-No. 37351, wo ein Extrakt von 50:50 in wässrigem Äthanol nach Verdampfen des Äthanols in Dosen von 40 ml/kg verabreicht wird; ebenso Biol. Abstracts, Vol. 87 (3), Ref.-No. 32970; ihr Einsatz gegen das oben beschriebene Syndrom ist aber in Biol. Abstracts, 91 (11), Ref-No. 123003 beschrieben]. Auch hier stellt die diuretische Wirkung eine Nebenerscheinung dar, die nicht unbedingt erwünscht ist. Durch die Kombination der beiden Pflanzenextrakte ergibt sich aber eine konzentrierte Wirkung im Hinblick auf die Syphilisbeschwerden, hingegen abgeschwächte Nebenwirkungen.

Dies trifft besonders zu, wenn entsprechend Anspruch 2 auch noch das Scheidenblatt benutzt wird, von dem etwa 36 Arten existieren, die meisten davon in den Tropen Amerikas. Nur zwei Arten finden sich im indo-malaysischen Inselarchipel, darunter Spatiphyllum commutatum auf den Philippinen. Für den Extrakt werden der Stamm und die Blätter verwendet. Spatiphyllum commutatum ist die drittwichtigste Pflanze im Rahmen der ins Auge gefassten Therapie.Ihre Anwendung für den vorliegenden Zweck wurde noch nirgends beschrieben oder nahegelegt.

Daneben können noch die folgenden Pflanzen, alle im Sinne einer Konzentration auf den angestrebten Zweck bei möglichst geringen Nebenwirkungen, eingesetzt werden. Sie werden alle mit dem Stamm, den Blüten und den Blättern zur Zubereitung des Extrakts verwendet.

Es handelt sich im wesentlichen um Bananengewächse, Musaceae, wobei die Anzahl der verwendeten Arten keine Rolle spielt:
Heliconia psittacorum, Papageienheliconie
Heliconia rostrata, geschnabelte Heliconie
Heliconia wagneriana, wagner'sche Heliconie
Strelitzia regina, Paradiesvogelblume
Strelitzia nicolai, weissblütige Strelitzie
Roter, weisser oder gelber Ingwer.

Die letztere Pflanze ist ja eher der Hedychie verwandt. Daneben ist aber auch noch der Kalebassenbaum, Crescentia cujete, einsetzbar. Die Gattung Crescentia ist in den Tropen der USA bis Florida verbreitet. Am bekanntesten ist C. cujete. Wie bereits erwähnt, werden von dieser letzteren Reihe anwendbarer bzw. zumischbarer Extrakte der Stamm und die Blätter verwendet, hier in diesem letzteren Falle auch die kürbisartigen Früchte. Wie im Falle des Calotropisextraktes ist eine gesonderte Anfertigung von Extrakten aus den einzelnen Pflanzenteilen zweckmässig.

### Beschreibung der Behandlung

Zunächst wurde ein Extrakt aus Blumen der Bananengewächse, wie sie oben angegeben sind, für einen langsamen Beginn der Behandlung angewandt. Zugleich wurde, separat, ein Mischextrakt aus Spatiphyllum hybridum und Hedychium coronarium verabreicht.

Im konkreten Falle war offenbar der Magen-Darm-Trakt besonders in Mitleidenschaft gezogen, so dass auf diese Behandlung ein Stuhlabgang einer gelblichen, übel riechenden Masse begann.

Sofort anschliessend wurde die Behandlungstinktur gewechselt und der Extrakt von Calotropis procera verabreicht. Dieser Extrakt ist ja, wie eingangs erwähnt, in der Medizin durchaus bekannt. Je nach der Dosierung ergab sich auch hier wiederum eine übel riechende gelbliche Ausscheidung über den Darm.

Ist dieser Erfolg eingetreten, so wird es angezeigt sein, die Behandlungstinktur ein weiteres Mal zu wechseln. Im konkreten Falle wurde Extrakt aus dem Kürbis der Crescentia verabreicht. Damit wurde eine Beruhigung innerhalb des Magen-Darm-Traktes erzielt, und die Ausscheidungen über den Darm normalisierten sich. Anschliessend wurde wieder mit der Blumentinkuren der Bananengewächse begonnen.

Wenn hier der Fall eines eher vorsichtig zu behandelnden Patienten geschildert wird, so sei erwähnt, dass die Reaktion bei eher robusteren Personen schwarzer Hautfarbe oder bei Patienten im Anfangsstadium der Krankheit viel weniger Anlass zu Bedenken gibt, so dass gegebenenfalls zuerst mit dem Extrakt von Calotropis angefangen werden kann. Dabei kann es auch zu Flüssigkeitsausscheidungen, aber auch zu einem Anschwellen der Füsse kommen. Wie erwähnt, lassen sich die Nebenwirkungen durch Wechsel der Extrakte in Grenzen zu halten. Fussbäder, z.B. in Flusswasser, lindern zusätzlich das Anschwellen der Füsse, wobei sich bei längeren Bädern ein weisser Rand bildet, so dass der Haut ebenfalls Aufmerksamkeit zu schenken ist, zumal ja, bereits krankheitsbedingt, mit Ekzemen, insbesondere juckenden Ekzemen zu rechnen ist.

Bei Patienten mit bereits länger anhaltender Krankheitsdauer, im konkreten ging es um einen Patienten mit einer Krankheitsdauer von annähernd 7 Jahren, ist eine intensivere Behandlung geboten, d.h. es empfiehlt sich mit dem Extrakt von ausgereifter Calotropis procera zu beginnen und mit dem Extrakter bereits älterer Blätter relativ intensiv fortzufahren. Sodann wird in der oben beschriebenen Weise auf Tinkturen von Hedychium coronarium und Spatiphyllum umgestellt. Am Ende erfolgte dann die Verabreichung von Extrakt der den Heliconien und Strelitzien und schliesslich die der übrigen Blütenextrakte.

Allgemein erhielten alle Patienten vitaminreiche Kost, zumal die Ermüdungserscheinungen unübersehbar waren.

### Dauer der Behandlung

Durch die Anwendung des Extraktes von Calotropis procera wird die Heilung beschleunigt, wobei der Allgemeinzustand des Patienten unter Kontrolle zu halten ist. Je nach Stadium der Erkrankung und Alter des Patienten kann aber eine Behandlung über bis zu 30 Monate erforderlich sein, wobei in der oben geschilderten Weise mit den Extrakten zweckmässig abgewechselt wird. Die Behandlung wird sich aber über wenigstens 3 Monate erstrecken.

### Dosierung

Die Dosierung richtet sich nach der Natur des Patienten, interessanterweise aber auch nach der Hautfarbe. Es wurde festgestellt, dass Personen schwarzer Hautfarbe ohne weiteres eine höhere Dosis als Weissen verabreicht werden kann. Im übrigen entspricht die Dosierung dem bereits bekannten Stande der Technik. Insgesamt werden bis zur Heilung etwa 40 bis 48 Liter Extrakt benötigt, d.h. etwa 5 bis 6 Liter jeder einzelnen Pflanze.

### Herstellung der Extrakte

Obwohl die Zubereitung der Extrakte zum grössten Teil bereits aus der Literatur vorbekannt ist, soll hier im einzelnen, und insbesondere auf die Besonderheiten im vorliegenden Falle eingegangen werden. Jede der Pflanzen (Frischpflanzen) dieser Zusammensetzung wird auf etwa 50 mm Länge kleingehackt. Im einzelnen wird auf die Pharmakopoe H.VII verwiesen.

| | |
|---|---|
| Calotropis procera: (Stamm, Samen und Blüten, ebenso alte Blätter) | 2 Teile der geschnittenen Droge + 8 Teile Äthanol (70%). Mazeration bei Raumtemperatur über etwa 1 Woche, dann Abfiltration und Abpressen. |
| Hedychium spec.: (Wurzeln) | wie oben, das Abpressen entfält. |
| Spatiphyllum + Hedychium: (Stamm und Blätter) | 1 Teil der geschnittenen Pflanze + 9 Teile Alkohol (70%), sonst wie oben. |
| Bananengewächse: (Heliconien + Strelitzien) | Vorgehen wie bei Spatiphyllum + Hedychium |
| Crescentia cujete: | Zubereitung wie Calotropis procera, ohne Abpressen |

Die obige Behandlung mit den Extrakten wurde zur Heilung angeborener Syphilis beschrieben, jedoch ist zu vermuten, dass die Extrakte bzw. besonders der Extrakt von Calotropis procera gegen Amöbenerkrankungen eingesetzt werden können.

## Patentansprüche

1. Verwendung von Calotropis procera für die Herstellung eines Extraktes zur Behandlung angeborener Syphilis.

2. Verwendung von Calotropis procera, Spatiphyllum hybridum und von Pflanzen der Gattung Hedychium für die Herstellung eines Extraktes zur Behandlung angeborener Syphilis.

3. Verwendung von Calotropis procera, Spatiphyllum hybridum und Pflanzen der Gattung Hedychium sowie wenigstens einer der folgenden Pflanzen:
- Heliconia psittacorum
- Heliconia rostrata
- Heliconia wagneriana
- Alpina purpurata
- Strelitzia reginae
- Strelitzia nicolai und/oder
- aus der Gattung Crescentia
für die Herstellung eines Extraktes zur Behandlung angeborener Syphilis.

## Claims

1. The use of Calotropis procera for producing an extract for the treatment of inherited syphilis.

2. The use of Calotropis procera, Spatiphyllum hybridum and plants of the Hedychium variety for producing an extract for the treatment of inherited syphilis.

3. The use of Calotropis procera, Spatiphyllum hybridum and plants of the Hedychium variety as well as at least one of the following plants:
- Heliconia psittacorum
- Heliconia rostrata
- Heliconia wagneriana
- Alpina purpurata
- Strelitzia reginae
- Strelitzia nicolai, and/or
- of the Crescentia variety
for producing an extract for the treatment of inherited syphilis.

## Revendications

1. Utilisation du Calotropis procera pour la préparation d'un extrait destiné au traitement de la syphilis congénitale.

2. Utilisation de Calotropis procera, Spatiphyllum hybridum et de plantes du type Hedychium, pour la fabrication d'un extrait destiné au traitement de la syphilis congénitale.

3. Utilisation de Calotropis procera, Spatiphyllum hybridum et de plantes du type Hedychium, ainsi que d'au moins l'une des plantes ci-après :
- Heliconia psittacorum
- Heliconia rostrata
- Heliconia wagneriana
- Alpina purpurata
- Strelitzia reginae
- Strelitzia nicolai et/ou
- du type Crescentia
pour la préparation d'un extrait pour le traitement de la syphilis congénitale.
